# DEMANDE DE BREVET EUROPEEN

(11) **EP 4 640 257 A1**
(43) Date de publication de la demande: **29.10.2025**
(21) Numéro de dépôt: 25168329.8
(22) Date de dépôt: 03.04.2025
(51) Int. Cl.: A61M 16/00, A61M 16/12, A61M 16/20

(54) **INSTALLATION DE FOURNITURE DE NO COMPRENANT UN APPAREIL DE DÉLIVRANCE DE NO ALIMENTÉ PAR DES BOUTEILLES DE GAZ**

(30) Priorité: 24.04.2024 FR 2404233
(71) Demandeur: INOSYSTEMS, 92160 Antony (FR)
(72) Inventeur: BLANDIN, Yann, 92160 Antony (FR); MARCHAL, Frederic, 92160 Antony (FR); TIRILLY, Nicolas, 92160 Antony (FR); PROUVEZ, Nathan, 92106 Antony (FR)
(74) Mandataire: Air Liquide

(57) **Abrégé**

Installation (100) de fourniture d'un mélange gazeux contenant du NO à un patient comprenant des récipients de NO (10) et un appareil de délivrance de NO (1) comprenant des moyens de pilotage (210), un circuit de gaz avec des tronçons de circuit (201, 202) comprenant des entrées de gaz (201.1, 202.1) pour y raccorder fluidiquement les récipients, ainsi que des moyens de mesure de pression (251, 252) et des moyens à vanne (222.1, 222.2) piloté par les moyens de pilotage pour autoriser une circulation de gaz dans l'un des tronçons et simultanément interdire toute circulation de gaz dans l'autre des tronçons tant que la pression mesurée est supérieure ou égale à une pression-seuil donnée, puis basculer d'un tronçon à l'autre lorsque la pression devient inférieure à la pression-seuil donnée.

## Description

L'invention concerne une installation de fourniture d'un mélange gazeux à base de NO à un patient comprenant un appareil de délivrance de NO pour fournir un mélange gazeux contenant du NO, typiquement un mélange NO/N₂, provenant d'une ou plusieurs sources de NO, telles des bouteilles de gaz sous pression, et un ventilateur médical fournissant un gaz à base d'oxygène (i.e. >20%vol. environ), tel de l'air ou un mélange O₂/N₂, de manière à obtenir un gaz combiné contenant du NO et de l'oxygène.

Le monoxyde d'azote inhalé (NO ou NOi) est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Pour mettre en œuvre une thérapie par NO inhalé, on utilise une installation de fourniture de gaz, aussi appelée installation d'administration de NO, comprenant un appareil de délivrance de NO et un ventilateur médical, c'est-à-dire un appareil d'assistance respiratoire, alimentant un circuit patient.

L'appareil de délivrance de NO permet d'injecter un mélange gazeux à base de NO, typiquement un mélange NO/azote, dans le circuit patient alimenté par ailleurs en un flux gazeux contenant de l'oxygène (au moins environ 20% vol.), tel de l'air ou un mélange oxygène/azote (O₂/N₂), fourni par le ventilateur médical. Le circuit patient comprend en général un ou plusieurs conduits flexibles reliés fluidiquement à une interface respiratoire, telle une sonde d'intubation trachéale ou analogue, servant à délivrer au patient à traiter, un mélange gazeux thérapeutique contenant une quantité ou dose donnée de NO, c'est-à-dire une posologie, typiquement entre 5 et 40 ppmv de NO.

Une telle installation de fourniture de gaz est décrite par exemple par EP3821929. Ce type d'installation est utilisé en milieu hospitalier pour administrer le traitement par NO et ainsi soigner les patients ayant besoin d'inhaler du NO pour traiter leur hypertension artérielle pulmonaire. D'autres installations de ce type sont décrites par EP4209243, EP4241817, EP4241812 et EP4295882.

Le NO gazeux, typiquement un mélange gazeux NO/azote, alimentant l'appareil de délivrance de NO provient généralement d'un ou plusieurs récipients de gaz sous pression, c'est-à-dire une ou des bouteilles de gaz contenant le mélange gazeux NO/azote comprimé, aussi appelées « bouteille de NO ».

Afin d'éviter une interruption de traitement du patient lorsqu'une bouteille de NO est vide, c'est-à-dire pendant le temps nécessaire à son remplacement par une bouteille de NO pleine, il est recommandé de raccorder fluidiquement l'appareil de délivrance de NO à deux bouteilles de NO. Ainsi, lorsqu'une bouteille de NO est (quasi)vide, un personnel soignant peut actionner une vanne de basculement ou analogue pour couper l'alimentation provenant de la bouteille de NO vide et autoriser celle provenant de la bouteille de NO pleine de manière à garantir une continuité de l'alimentation fluidique en NO de l'appareil de délivrance de NO et à permettre le remplacement de la bouteille de NO vide par une pleine.

Un problème qui se pose en pratique est que les personnels soignants peuvent soit oublier de vérifier la pression affichée par la jauge de pression équipant habituellement une bouteille de NO et dès lors ne pas s'apercevoir qu'une bouteille est (quasi)vide, soit ne pas être disponibles pour réaliser ces opérations car trop occupés à traiter un patient. Dans les deux cas, il en résulte un risque de rupture de fourniture de NO, ce qui n'est pas acceptable car pouvant potentiellement mettre le patient en danger.

WO2015/172160 et US2023270960 proposent une gestion des alimentations en NO gazeux provenant de bouteilles de NO d'une installation de fourniture de NO, basée sur le calcul d'une autonomie en gaz, appelée run-time-to-empty, c'est-à-dire un temps d'utilisation avant vidange totale de chaque bouteille. Le calcul se fait à partir d'un suivi du niveau de pression résiduel dans les bouteilles de NO. Cette manière de procéder n'est pas idéale car elle requiert de mesurer en permanence la pression dans les bouteilles de gaz elles-mêmes, ce qui complexifie l'architecture globale de l'installation.

Au vu de cela, un but de l'invention est de pouvoir éviter ou minimiser ce risque de rupture de fourniture de NO engendré par un non-remplacement d'une bouteille de NO (quasi)vide d'une installation de fourniture d'un mélange gazeux à base de NO à un patient de manière à améliorer la sécurité de traitement d'un patient traité par administration de NO gazeux.

Une solution de l'invention concerne une installation de fourniture d'un mélange gazeux contenant du NO à un patient comprenant :
A) un premier et un second récipient de NO, telles des bouteilles de gaz, contenant un gaz contenant du NO à une pression donnée,
B) un appareil ou dispositif de délivrance, i.e. fourniture, de NO pour fournir le gaz contenant du NO, typiquement un mélange NO/N₂, comprenant :
   - des moyens de pilotage, de préférence à microprocesseur, tel un contrôleur électronique ou analogue,
   - un circuit de gaz comprenant :
      ▪ un premier tronçon de circuit comprenant une première entrée de gaz configurée pour y raccorder fluidiquement le premier récipient de NO contenant le gaz contenant du NO, et
      ▪ un second tronçon de circuit comprenant une seconde entrée de gaz configurée pour y raccorder fluidiquement le second récipient de NO contenant le gaz contenant du NO,
   - un premier moyen de mesure de pression, agencé sur le premier tronçon de circuit, configuré pour mesurer la pression au sein du premier tronçon de circuit,
   - un second moyen de mesure de pression, agencé sur le second tronçon de circuit, configuré pour mesurer la pression au sein du second tronçon de circuit,
   - un premier moyen à vanne, agencé sur le premier tronçon de circuit, piloté par les moyens de pilotage pour contrôler le flux de gaz au sein du premier tronçon de circuit, c'est-à-dire autoriser/permettre ou, à l'inverse, interdire/stopper toute circulation de gaz au sein du premier tronçon de circuit et
   - un second moyen à vanne, agencé sur le second tronçon de circuit, piloté par les moyens de pilotage pour contrôler le flux de gaz au sein du second tronçon de circuit, c'est-à-dire autoriser/permettre ou, à l'inverse, interdire/stopper toute circulation de gaz au sein du second tronçon de circuit,
C) des premier et second moyens de détente agencés en amont de l'appareil de délivrance de NO et configurés pour opérer une réduction de pression du gaz contenant du NO provenant des premier et second récipient de NO jusqu'à une pression de détente (PD) donnée inférieure à 10 bar, et
D) une première ligne d'amenée de gaz et une seconde ligne d'amenée de gaz, respectivement, pour acheminer le gaz contenant du NO ayant traversé les moyens de détente (i.e. les premier et second moyens de détente) jusqu'à la première entrée de gaz du premier tronçon de circuit et à la second entrée de gaz du second tronçon de circuit, respectivement.

De plus, les moyens de pilotage de l'appareil de délivrance de NO d'une installation selon l'invention sont configurés pour piloter le premier moyen à vanne et/ou le second moyen à vanne pour :
i) autoriser une circulation de gaz au sein de l'un des premier et second tronçons de circuit et simultanément interdire toute circulation de gaz au sein de l'autre desdits premier et second tronçons de circuit tant que la pression (P) mesurée (e.g. la pression instantanée) par le premier ou le second moyen de mesure de pression est supérieure ou égale à une pression-seuil donnée (PS), i.e. P ≥ PS, et
ii) lorsque la pression mesurée (P) par le premier ou le second moyen de mesure de pression devient inférieure à la pression-seuil donnée (PS), i.e. P < PS :
   a) interrompre toute circulation de gaz au sein dudit premier ou second tronçon de circuit au sein duquel du gaz circule, c'est-à-dire dans l'un des deux tronçons, et
   b) autoriser une circulation de gaz au sein de l'autre desdits premier ou second tronçon de circuit au sein duquel la circulation de gaz était interdite, c'est-à-dire dans l'autre des deux tronçons,
où ladite pression-seuil donnée (PS) est inférieure à la pression de détente (PD), c'est-à-dire PS < PD.

Autrement dit, dans l'appareil de l'invention, il s'opère un basculement automatique d'un tronçon vers l'autre, dès lors que la pression mesurée au sein du tronçon considéré devient inférieure ou égale à la pression-seuil (PS) donnée, ce qui correspond à une bouteille de gaz vide ou quasi-vide, en particulier lorsque la pression mesurée devient inférieure à une pression-seuil (PS) de l'ordre de 3 à 4 bar.

L'alimentation en gaz se fait donc de manière alternative, en fonction de la pression régnant dans les premier et second tronçons de circuit.

Ainsi, les moyens de pilotage de l'appareil de délivrance de NO sont configurés pour piloter le premier moyen à vanne et/ou le second moyen à vanne pour :
- autoriser/permettre une circulation de gaz au sein du premier tronçon de circuit et simultanément interdire toute circulation de gaz au sein du second tronçon de circuit tant que la pression (P) mesurée par le premier moyen de mesure de pression est supérieure ou égale à la pression-seuil donnée (PS),
- puis, lorsque la pression mesurée (P) par le premier moyen de mesure de pression devient inférieure à la pression-seuil donnée (PS), pour interrompre/stopper toute circulation de gaz au sein dudit premier tronçon de circuit au sein duquel du gaz circulait jusqu'alors, et autoriser ensuite une circulation de gaz au sein du second tronçon de circuit au sein duquel la circulation de gaz était interdite/empêchée jusqu'alors

Et ensuite, réciproquement ou alternativement, les moyens de pilotage de l'appareil de délivrance de NO sont configurés pour piloter le premier moyen à vanne et/ou le second moyen à vanne pour :
- autoriser/permettre une circulation de gaz au sein du second tronçon de circuit et simultanément interdire toute circulation de gaz au sein du premier tronçon de circuit tant que la pression (P) mesurée par le second moyen de mesure de pression est supérieure ou égale à la pression-seuil donnée (PS),
- puis, lorsque la pression mesurée (P) par le second moyen de mesure de pression devient inférieure à la pression-seuil donnée (PS), pour interrompre/stopper toute circulation de gaz au sein dudit second tronçon de circuit au sein duquel du gaz circulait, et autoriser ensuite une circulation de gaz au sein du premier tronçon de circuit au sein duquel la circulation de gaz était interdite/empêchée.

Dit encore autrement, l'appareil ou dispositif de délivrance, i.e. fourniture, de NO servant à fournir un gaz contenant du NO dans une installation selon l'invention comprend:
- des moyens de pilotage, et
- un circuit de gaz comprenant au moins deux tronçons de circuit agencés, de préférence en parallèle, comprenant chacun :
   ∘ une entrée de gaz configurée pour y raccorder fluidiquement un récipient de NO contenant le gaz contenant du NO, et
   ∘ un moyen de mesure de pression configuré pour y mesurer la pression gazeux qui y règne, et
   ∘ un moyen à vanne piloté par les moyens de pilotage pour y contrôler le flux de gaz, c'est-à-dire autoriser/permettre ou, à l'inverse, interdire/stopper toute circulation de gaz au sein du tronçon considéré,
et dans lequel les moyens de pilotage sont configurés pour piloter l'un des moyens à vanne pour :
- autoriser une circulation de gaz au sein de l'un des tronçons et simultanément interdire toute circulation de gaz au sein de l'autre desdits tronçons tant que la pression (P) mesurée par le moyen de mesure de pression associé au tronçon considéré est supérieure ou égale à une pression-seuil donnée (PS), i.e. P ≥ PS,
- et lorsque la pression mesurée (P) devient inférieure à la pression-seuil donnée (PS), sein du tronçon considéré :
   a) interrompre toute circulation de gaz au sein du tronçon considéré au sein duquel du gaz circulait, i.e. où un flux passait jusqu'à présent, et
   b) autoriser une circulation de gaz au sein de l'autre tronçon au sein duquel la circulation de gaz était interdite, c'est-à-dire dans l'autre des tronçons, i.e. le tronçon où le flux gazeux était interrompu jusqu'à présent.

Avantageusement, avant d'autoriser une circulation de gaz au sein de l'autre tronçon, une purge dudit tronçon est opérée.

Selon le mode de réalisation considéré, l'installation de mélange gazeux contenant du NO selon l'invention peut comprendre l'une ou plusieurs des caractéristiques suivantes :
- le premier tronçon de circuit et le second tronçon de circuit de l'appareil de délivrance de NO, typiquement de mélange NO/N₂, viennent se raccorder l'un à l'autre en un site de raccordement du circuit de gaz situé en aval des premier et second moyens à vanne.
- les premier et second moyens à vanne de l'appareil de délivrance de NO comprennent des électrovannes.
- la première entrée de gaz de l'appareil de délivrance de NO est configurée pour être raccordée fluidiquement au premier récipient de NO par l'intermédiaire d'une première ligne d'amenée de gaz.
- la seconde entrée de gaz de l'appareil de délivrance de NO est configurée pour être raccordée fluidiquement au second récipient de NO par l'intermédiaire d'une seconde ligne d'amenée de gaz.
- la première ligne d'amenée de gaz et la seconde ligne d'amenée de gaz comprennent des tuyaux flexibles ou similaires.
- des premiers moyens de réduction de pression sont agencés en amont de la première entrée de gaz du premier tronçon de circuit.
- des seconds moyens de réduction de pression sont agencés en amont de la seconde entrée de gaz du second tronçon de circuit.
- les premiers moyens de réduction de pression et/ou les seconds moyens de réduction de pression comprennent au moins un dispositif détendeur de gaz, c'est-à-dire un dispositif réducteur de pression configuré pour réduire, i.e. diminuer, la pression du gaz qui le traverse, à savoir ici la pression du mélange gazeux à base de NO.
- les premiers moyens de réduction de pression et/ou les seconds moyens de réduction de pression sont configurés pour réduire (i.e. contrôler) la pression du gaz contenant du NO jusqu'à une pression de détente (PD) inférieure à 10 bar, typiquement de l'ordre de 4 à 8 bar, de préférence entre 4 et 7 bar, de préférence encore entre 4 et 6 bar, avantageusement entre 5 et 6 bar.
- les premiers moyens de réduction de pression sont agencés en aval du premier récipient de NO contenant le gaz à base de NO, typiquement un mélange NO/N₂.
- les seconds moyens de réduction de pression sont agencés en aval du second récipient de NO contenant le gaz à base de NO, typiquement un mélange NO/N₂.
- lorsque les récipients de NO sont pleins, le mélange NO/N₂ y est conditionné à une pression (i.e. une pression de départ) d'au moins 150 bar appelée « haute pression ». Cette haute pression correspond à la pression du gaz à base de NO avant détente, e.g. mélange NO/N₂, c'est-à-dire avant réduction de pression par passage dans les premiers et/ou les seconds moyens de réduction de pression.
- le premier récipient de gaz est équipé d'un premier robinet de distribution de gaz servant à contrôler la fourniture de gaz à base de NO provenant du premier récipient de gaz, c'est-à-dire la distribution de gaz.
- le second récipient de gaz est équipé d'un second robinet de distribution de gaz servant à contrôler la fourniture de gaz à base de NO provenant du second récipient de gaz, c'est-à-dire la distribution de gaz.
- selon un mode de réalisation, les premiers et/ou les seconds moyens de réduction de pression sont agencés en aval, i.e. en sortie, des premier et second robinets de distribution agencés sur les premier et second récipients de gaz, respectivement.
- selon un autre mode de réalisation, les premiers et/ou les seconds moyens de réduction de pression sont intégrés aux robinets de distribution agencés sur les premier et second récipients de gaz, respectivement, c'est-à-dire que lesdits robinets de distribution de gaz sont des robinets à détendeurs intégrés (RDI).
- la première entrée de gaz de l'appareil est configurée pour être raccordée fluidiquement au premier robinet du premier récipient de NO par l'intermédiaire de la première ligne d'amenée de gaz.
- la seconde entrée de gaz de l'appareil est configurée pour être raccordée fluidiquement au second robinet du second récipient de NO par l'intermédiaire de la seconde ligne d'amenée de gaz.
- les premiers moyens de réduction de pression sont agencés entre une sortie de gaz du premier robinet du premier récipient de NO et une entrée de gaz de la première ligne d'amenée de gaz.
- les seconds moyens de réduction de pression sont agencés entre une sortie de gaz du second robinet du second récipient de NO et une entrée de gaz de la seconde ligne d'amenée de gaz.
- le gaz à base de NO subit une diminution de sa pression jusqu'à la pression de détente (PD), en passant dans, i.e. en traversant, les premiers moyens de réduction de pression ou les seconds moyens de réduction de pression.
- le gaz à base de NO circule dans la première et la seconde ligne d'amenée de gaz dans le sens allant des récipients de NO vers l'appareil de délivrance, i.e. en direction des première et seconde entrées de gaz.
- le gaz à base de NO amené par la première et/ou la seconde ligne d'amenée de gaz est à la pression de détente (PD).
- l'appareil comprend en outre une première ligne d'échappement à l'atmosphère comprenant une première valve d'échappement, raccordée fluidiquement au premier tronçon de circuit.
- l'appareil comprend en outre une seconde ligne d'échappement à l'atmosphère comprenant une seconde valve d'échappement, raccordée fluidiquement au second tronçon de circuit.
- les première et seconde valves d'échappement de l'appareil sont pilotées par les moyens de pilotage.
- la première ligne d'échappement à l'atmosphère et la seconde ligne d'échappement à l'atmosphère communiquent avec l'atmosphère via un ou plusieurs orifices d'échappement de l'appareil.
- l'échappement du gaz vers l'atmosphère ambiante se fait via un orifice d'échappement unique ou alternativement via plusieurs orifices d'échappement de l'appareil, par exemple deux orifices d'échappement.
- les moyens de pilotage de l'appareil sont en outre configurés pour piloter la seconde valve d'échappement pour opérer une purge du second tronçon de circuit, avant d'autoriser une circulation de gaz provenant du second récipient de NO au sein dudit second tronçon de circuit.
- alternativement, les moyens de pilotage de l'appareil sont en outre configurés pour piloter la première valve d'échappement pour opérer une purge du premier tronçon de circuit, avant d'autoriser une circulation de gaz provenant du premier récipient de NO au sein dudit premier tronçon de circuit.
- les moyens de pilotage de l'appareil sont en outre configurés pour opérer une purge du premier ou du second tronçon de circuit et simultanément d'au moins une partie de la première ou de la seconde ligne d'amenée de gaz reliée audit premier ou second tronçon de circuit soumis à purge, c'est-à-dire un ou des tuyaux flexibles.
- les moyens de pilotage sont en outre configurés pour opérer une purge entre les étapes a) et b) ci-dessus.
- les moyens de pilotage sont en outre configurés pour opérer une purge pendant une durée de purge donnée.
- la durée de purge comprise entre 10 secondes et 120 secondes, typiquement d'au moins 30 secondes.
- la durée de purge est mémorisée, par exemple par des moyens de mémorisation de l'appareil.
- la purge du premier et/ou du second tronçon de circuit comprend un envoi ou rejet vers l'atmosphère d'au moins une partie du gaz sous pression (i.e. de la pression résiduelle) présent dans le premier ou le second tronçon de circuit de l'appareil et préférentiellement dans au moins une partie de la première ou de la seconde ligne d'amenée de gaz reliée audit premier ou second tronçon de circuit soumis à purge de l'appareil.
- la purge du premier et/ou du second tronçon de circuit comprend un envoi ou rejet vers l'atmosphère d'au moins une partie du gaz sous pression et au moins une partie des espèces non-désirées pouvant s'y trouver, en particulier les espèces néfastes ou toxiques, typiquement les espèces NO₂.
- la purge comprend un balayage gazeux avec le mélange gazeux contenant du NO, typiquement le mélange NO/N₂, provenant du premier ou du second récipient de NO.
- la purge du premier et/ou du second tronçon de circuit comprend un balayage gazeux (au moins) dudit premier et/ou second tronçon de circuit avec le mélange gazeux NO/N₂, i.e. le gaz à base de NO provenant de l'un ou l'autre des récipients de gaz.
- la purge comprend un balayage gazeux dudit premier ou second tronçon de circuit, respectivement et de ladite première ou de la seconde ligne d'amenée de gaz, respectivement, i.e. un balayage gazeux du premier tronçon et de la première ligne d'amenée de gaz qui lui est associée ou du second tronçon et de la seconde ligne d'amenée de gaz qui lui est associée, avec le mélange gazeux NO/N₂, i.e. le gaz à base de NO provenant de l'un ou l'autre des récipients de gaz, i.e. le premier ou second récipient de gaz, respectivement.
- un envoi ou rejet vers l'atmosphère d'au moins une partie de l'atmosphère gazeuse (i.e. gaz contenant des impuretés NO₂ éventuelles) présente dans le premier ou le second tronçon de circuit de l'appareil et dans la première ou de la seconde ligne d'amenée de gaz, à savoir du gaz de purge contenant le mélange NO/N₂ ayant servi au balayage gazeux et éventuellement des impuretés, tels les espèces NO₂.
- la pression-seuil donnée (PS) est mémorisée par des moyens de mémorisation de l'appareil.
- la pression-seuil donnée (PS) est comprise entre 2 et 5 bar, de préférence entre 2 et 4 bar, préférentiellement encore entre 3 et 4 bar.
- le premier récipient de NO et le second récipient de NO contiennent un mélange NO/N₂ contenant entre 100 et 2000 ppmv de NO, et de l'azote pour le reste.
- le premier récipient de NO et le second récipient de NO contiennent un mélange NO/N₂ contenant entre 100 et 1500 ppmv, typiquement entre 200 et 1000 ppmv.
- l'appareil est alimenté en un mélange gazeux formé d'azote et de NO.
- l'appareil comprend des moyens de réglage de dose configurés pour permettre à un utilisateur de fixer ou sélectionner la consigne de teneur en NO correspondant à la proportion finale de NO souhaitée dans le mélange gazeux combiné, i.e. une posologie.
- les moyens de réglage de dose font partie d'une IHM (interface homme-machine) ou IGU (interface graphique utilisateur) de l'appareil.
- les moyens de réglage de dose de l'appareil comprennent une ou des touches tactiles, actionnables par l'utilisateur, s'affichant sur un écran digital à dalle tactile de l'IHM, de préférence du type à affichage en couleurs.
- la consigne de teneur en NO est comprise entre 1 et 80 ppmv, typiquement entre 5 et 40 ppmv.
- les moyens de mémorisation de l'appareil comprennent une mémoire informatique, telle une mémoire flash, une RAM ou analogue.
- les moyens de pilotage comprennent un (micro)contrôleur ou analogue.
- les moyens de pilotage comprennent un (ou des) (micro)processeur agencé sur une (ou des) carte électronique.
- les moyens de pilotage comprennent un (ou des) (micro)processeur mettant en œuvre un ou des algorithmes, notamment un (ou des) algorithme de pilotage ou de contrôle des vannes ou valves, de traitement des mesures de débit ou de pression....
- les moyens de mémorisation sont agencés sur la carte électronique.
- il est alimenté électriquement par une ou des sources de courant électrique, typiquement le secteur (110/220V) et/ou une ou des batteries rechargeables.
- l'appareil de délivrance de NO est alimenté en un gaz contenant du NO provenant de deux récipients de gaz alimentant alternativement l'un ou l'autre des tronçons du circuit de gaz de l'appareil, typiquement deux bouteilles de gaz sous pression.
- l'installation comprend un ventilateur médical configuré pour fournir un flux de gaz respiratoire contenant de l'O₂, tel de l'air ou un mélange O₂/N₂.
- l'installation comprend un circuit respiratoire comprenant un dispositif d'injection configuré pour opérer un mélange du gaz contenant du NO provenant de l'appareil de délivrance de NO avec le flux de gaz respiratoire contenant de l'O₂ fourni par le ventilateur médical, et obtenir un mélange gazeux combiné contenant du NO et de l'oxygène.
- l'appareil de délivrance de NO et le ventilateur médical sont raccordés fluidiquement au dispositif d'injection agencé sur le circuit respiratoire.
- le ventilateur médical est configuré pour fournir un flux de gaz respiratoire contenant au moins 20%vol. environ d'O₂, typiquement un mélange NO/N₂ ou de l'air.
- l'appareil de délivrance de NO et le ventilateur médical sont raccordés fluidiquement au circuit respiratoire, en particulier via le dispositif d'injection.
- un capteur de débit est agencé dans le circuit respiratoire entre le ventilateur médical et le dispositif d'injection.
- le dispositif d'injection comprend une première entrée de gaz alimentée en flux de gaz respiratoire contenant de l'O₂, i.e. provenant du ventilateur médical.
- le dispositif d'injection comprend en outre une seconde entrée de gaz alimentée en gaz contenant du NO audit débit de consigne, i.e. provenant de l'appareil de délivrance de NO.
- le dispositif d'injection comprend en outre une sortie de gaz fournissant le mélange gazeux combiné contenant du NO et de l'oxygène, obtenu par mélange, au sein du dispositif d'injection, du gaz contenant du NO (e.g. mélange NO/N₂) avec le flux de gaz respiratoire contenant de l'O₂ (e.g. air ou mélange O₂/N₂).
- le ventilateur médical délivre de l'air ou un mélange oxygène/azote, i.e. en tant que gaz respiratoire contenant au moins 20% vol. environ d'oxygène, de préférence au moins 21% vol. environ d'oxygène.
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- chaque récipient de NO contient un mélange gazeux NO/N₂ contenant entre 100 et 2000 ppmv de NO, le reste étant de l'azote (N₂), de préférence entre 100 et 1000 ppmv de NO.
- chaque récipient de NO contient un mélange gazeux NO/N₂ conditionné à une pression (mesurée avant début de soutirage) comprise entre 10 et 250 bar, de préférence plus de 50 bar, typiquement à plus de 100 bar.
- chaque récipient de NO est ou comprend une (ou des) bouteille de gaz ayant une contenance comprise entre 0,5 et 50 L (équivalent en eau).
- chaque récipient de NO comprend un corps cylindrique en acier ou en alliage d'aluminium.
- chaque récipient de NO est équipé d'un robinet de distribution de gaz dit « simple » (sans détendeur intégré) ou d'un robinet dit « à détendeur intégré » (RDI) intégrant des moyens de détente (i.e. de réduction de pression), de préférence un RDI.
- chaque robinet de distribution de gaz (i.e. simple ou RDI) est protégé par un capotage de protection, par exemple en métal ou polymère.
- chaque récipient de NO est équipé d'un robinet « simple » (i.e. sans détendeur intégré) et un dispositif détendeur de gaz est agencé en aval de chaque robinet.
- les moyens de réduction de pression, i.e. les moyens détente de gaz, sont agencés en aval du robinet « simple » de chaque récipient de NO contenant le gaz à base de NO, typiquement un mélange NO/N₂ de manière à opérer une diminution ou réduction de pression du gaz à base de NO, typiquement un mélange NO/N₂, sortant du robinet considéré, i.e. de chaque robinet, et obtenir ainsi le gaz à la pression de détente (PD) souhaitée inférieure à 10 bar.
- les moyens de réduction de pression comprennent ou sont (au moins) un dispositif détendeur de gaz, plus simplement appelé « détendeur ».
- chaque détendeur est fixé à un robinet (simple) en étant raccordé fluidiquement à la sortie dudit robinet, par exemple par vissage ou analogue.
- alternativement, les moyens de réduction de pression sont agencés dans chaque robinet équipant chaque récipient de NO, c'est-à-dire intégrés à chaque robinet de type RDI, de manière à opérer une diminution ou réduction de pression du gaz à base de NO, typiquement un mélange NO/N₂, avant sa sortie du robinet considéré, i.e. de chaque robinet.
- dans tous les cas, les moyens de réduction de pression sont configurés pour réduire la pression du gaz à base de NO, typiquement un mélange NO/N₂, jusqu'à une pression de détente inférieure ou égale à 10 bar, de préférence inférieure à 8 bar, typiquement entre 4 et 7 bar, par exemple entre 5 et 6 bar.
- les lignes d'amenée de gaz, typiquement la première et la seconde ligne d'amenée de gaz, sont raccordées en aval de chaque RDI ou de chaque détendeur.
- les lignes d'amenée de gaz, typiquement la première et la seconde ligne d'amenée de gaz, sont configurées pour acheminer le gaz à base de NO après réduction de sa pression jusqu'à la pression de détente (PD) inférieure ou égale à 10 bar, c'est-à-dire le gaz détendu.
- les lignes d'amenée de gaz, typiquement la première et la seconde ligne d'amenée de gaz, comprennent des tuyaux flexibles, typiquement en polymère.
- la sortie de gaz de chaque robinet (simple ou RDI) est agencée sur un raccord de sortie, tel un embout ou analogue.
- le circuit respiratoire de l'installation comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire, ou autre.
- la branche inspiratoire et la branche expiratoire sont en outre fluidiquement raccordées à, respectivement, des orifices de sortie et d'entrée du ventilateur médical.
- le circuit respiratoire, en particulier la branche inspiratoire, peut comprendre un humidificateur de gaz.
- l'humidificateur de gaz est agencé en aval du dispositif d'injection, par exemple un module d'injection de NO, de manière à pouvoir humidifier le gaz avant son administration par inhalation au patient.
- le ventilateur médical est alimenté électriquement par une ou des sources de courant électrique, typiquement le secteur (110/220V) et/ou une ou des batteries rechargeables.

Selon un autre aspect, l'invention concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO assurant une délivrance de NO à la posologie souhaitée, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les pressions sont exprimées en bar absolus, abrévié « bar ».
- les termes « concentration », « quantité », « proportion », « dose » et « teneur » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue.
- les termes « amont » et « aval » sont utilisés par rapport au sens normal de circulation du gaz, à savoir dans le sens allant des récipients de gaz vers l'appareil de délivrance, puis vers le patient.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'administration de gaz selon l'invention.
Fig. 2 schématise un mode de réalisation de l'architecture interne de l'appareil de délivrance de NO d'une installation d'administration de gaz selon l'invention, en particulier une installation d'administration de gaz selon Fig. 1.

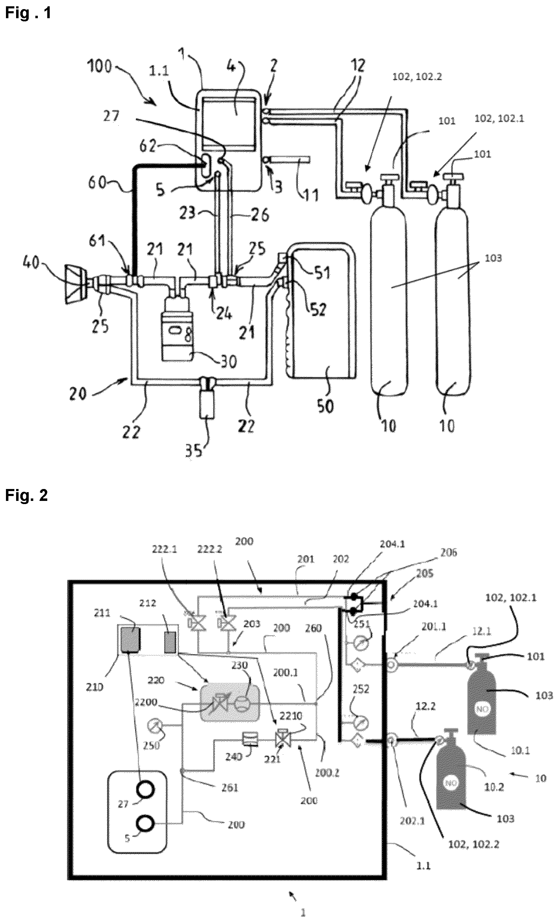

Fig. 1 schématise un mode de réalisation d'une installation d'administration de gaz 100 selon l'invention comprenant un appareil de fourniture de NO 1 fournissant un mélange gazeux à base de monoxyde d'azote (NO), et un ventilateur médical 50 fournissant un gaz contenant au moins 20%vol. d'oxygène, tel de l'air ou autre.

L'installation 100 comprend des sources de NO 10, à savoir ici deux récipients ou bouteilles de gaz sous pression 10.1, 10.2 contenant chacune un gaz à base de NO, c'est-à-dire un mélange gazeux NO/N₂, à savoir ici un mélange gazeux NO/N₂ contenant entre 100 et 1000 ppmv de NO (reste N₂), par exemple 450 ou 800 ppm vol. de NO (reste N₂), ou toute autre concentration adéquate, qui alimentent en mélange NO/N₂, le dispositif ou appareil 1 de délivrance ou fourniture de NO permettant de suivre et contrôler la fourniture du mélange gazeux NO/N₂.

Le mélange gazeux NO/N₂ est généralement conditionné dans chaque récipients 10.1, 10.2 à une pression, appelée « pression haute », d'au moins 150 bar, laquelle correspond à la pression de départ régnant dans chaque récipient avant le début de tout soutirage, c'est-à-dire mesurée avant tout soutirage ou toute utilisation de gaz (i.e. récipient plein). Dans certains cas, elle peut atteindre 230 à 250 bar, voire même plus. Bien entendu, la pression du gaz dans les récipients 10.1, 10.2 diminue au fur et à mesure du soutirage du gaz, c'est-à-dire de l'utilisation progressive du gaz, engendrant ainsi une vidange progressive des récipients 10.1, 10.2.

Comme illustré en Fig. 1, chaque récipient 10.1, 10.2 comprend un corps 103 de forme générale cylindrique ou d'ogive, contenant le gaz au sein de son volume interne, lequel corps 103 est surmonté d'un robinet 101 de distribution du gaz servant à contrôler la sortie du gaz du volume interne du corps 103.

Sur Fig. 1, chaque robinet 101 est un robinet dit « simple », c'est-à-dire ne contenant pas de moyens de détente interne. De ce fait, des moyens de détente 102 externes, à savoir des premier et second moyens de détente 102.1, 102.2, tels des détendeurs de gaz, sont agencés en aval de chaque robinet 101 afin d'assurer une réduction de la pression gazeuse, à une pression de détente (PD) souhaitée qui est réglée au niveau de chaque détendeur, typiquement une pression de détente inférieure ou égale à 10 bar, de préférence inférieure ou égale à 8 bar, typiquement entre 4 et 7 bar, avantageusement entre 5 et 6 bar. Les détendeurs de gaz peuvent être par exemple vissés sur la sortie de gaz des robinets 101.

Alternativement, selon un autre mode de réalisation (non montré), les robinets 101 pourraient être du type à détendeur intégré (RDI), c'est-à-dire que les moyens de détente 102 seraient alors agencés dans (i.e. intégrés) le corps de chaque robinet 101, par exemple un clapet de détente coopérant avec un siège de clapet. Dans ce cas, le gaz sortant de chaque robinet 101 serait alors à la pression de détente souhaitée, i.e. à une pression de détente inférieure ou égale à 10 bar, comme précédemment.

Les bouteilles de gaz 10.1, 10.2 sont chacune reliées fluidiquement à l'appareil 1 de fourniture de NO, via des lignes d'amenée de gaz 12, typiquement une première ligne d'amenée de gaz 12.1 et une seconde ligne d'amenée de gaz 12.2, tel que des tuyaux ou conduits flexibles ou analogues, qui peuvent être équipées de dispositifs de suivi de la pression de gaz, notamment un ou des manomètres ou autres.

Les lignes d'amenée de gaz 12 acheminent le gaz à base de NO à la pression de détente (PD), c'est-à-dire le gaz détendu au sein des moyens de détente 102, i.e. les premier 102.1 et second 102.2 moyens de réduction de pression, jusqu'à l'appareil 1.

Plus précisément, les lignes d'amenée de gaz 12 sont raccordées fluidiquement à des entrées de gaz 2, à savoir une premier et une seconde entrée de gaz 201.1, 202.1 de l'appareil de délivrance de NO 1, qui alimentent un circuit de gaz interne 200, comme détaillé en Fig. 2, servant à acheminer le gaz au sein de l'appareil de fourniture de NO 1, c'est-à-dire dans le boitier ou carcasse 1.1 externe de l'appareil 1.

Dans le mode de réalisation de Fig. 2, le circuit de gaz interne 200 comprend un premier tronçon de circuit 201, aussi appelé premier tronçon d'entrée, relié fluidiquement à une première entrée de gaz 201.1 et un second tronçon de circuit 202, aussi appelé second tronçon d'entrée, relié fluidiquement à une seconde entrée de gaz 202.1 de l'appareil 1. Les première et seconde entrées de gaz 201.1, 202.1 sont aussi appelées première et seconde entrées de NO puisqu'elles sont alimentées en mélange NO/N₂ provenant des bouteilles de gaz 10.1, 10.2.

Le premier tronçon de circuit 201 comprend un premier moyen à vanne 222.1, typiquement une première vanne de contrôle, piloté par les moyens de pilotage 210 pour contrôler le flux de gaz au sein du premier tronçon de circuit 201, et le second tronçon de circuit 202 comprend un second moyen à vanne 222.2, typiquement une seconde vanne de contrôle, piloté par les moyens de pilotage 210 pour contrôler le flux de gaz au sein du second tronçon de circuit 202, typiquement les premier et second moyens à vanne 222.1, 222.2 sont des électrovannes ou analogues.

Comme illustré en Fig. 2, les deux tronçons de circuit 201, 202 sont agencées en parallèle l'un de l'autre mais viennent se raccorder fluidiquement l'un à l'autre en un site de raccordement 203 du circuit de gaz 200, lequel site de raccordement 203 se trouve en aval des moyens à vannes 222.1, 222.2, en considérant le sens de circulation du gaz dans le circuit de gaz 200, c'est-à-dire dans le sens allant des première et seconde entrées de gaz 201.1, 202.1 vers les vannes de contrôle 222.1, 222.2.

La première entrée de gaz 201.1 est raccordée fluidiquement à la première ligne d'amenée de gaz 12.1, typiquement un premier tuyau flexible, alors que la seconde entrée de gaz 201.1 est raccordée fluidiquement à la seconde ligne d'amenée de gaz 12.2, typiquement un second tuyau flexible. Les raccordements peuvent être assurés par des connecteurs classiques.

D'une façon générale, la pression gazeuse qui règne dans les deux tronçons de circuit 201, 202 correspond à la pression gazeuse acheminée par les lignes d'amenée de gaz 12.1, 12.2, donc à la pression de détente tant que les récipients alimentant ces tronçons contiennent une quantité suffisante de gaz à base de NO.

En effet, chaque ligne d'amenée de gaz 12 contient et achemine du gaz à base de NO à une pression qui est d'abord égale à la pression de détente (PD), i.e. moins de 10 bar, par exemple entre 4 et 7 bar, puis qui devient inférieure à la pression de détente (PD) lorsque le récipient de NO qui alimente la ligne d'amenée de gaz 12 considérée est proche d'être vide, c'est-à-dire que la majeure partie (i.e. quasi totalité) du gaz qu'il contient a été utilisé.

En d'autres termes, le gaz à base de NO, qu'il soit à la pression de détente (PD) tant que le récipient fournissant le gaz à base de NO contient suffisamment de gaz ou à une pression inférieure à la pression de détente et qui continue à diminuer au fur et à mesure que le récipient considéré se vide, est fourni par l'une ou l'autre des lignes d'amenée de gaz 12 à l'un ou l'autre des deux tronçons de circuit 201, 202 de l'appareil 1.

La pression du gaz au sein au sein des deux tronçons de circuit 201, 202 est suivie en permanence, i.e. mesurée par, des moyens de mesure de pression, à savoir un premier et un second moyen de mesure de pression 251, 252, agencés sur les premier et second tronçons 201, 202 de circuit, respectivement, tels des capteurs de pression agencés de manière à pouvoir opérer des mesures de pression au sein des tronçons de circuit 201, 202 et fournir ces mesures aux moyens de pilotage 210, notamment afin de pouvoir opérer un basculement automatique d'un tronçon vers l'autre 201, 202, lorsque la pression mesurée au sein du tronçon considéré 201, 202 devient inférieure ou égale à la pression-seuil (PS) fixée, par exemple de l'ordre de 4 bar.

Ce basculement automatique est commandé par les moyens de pilotage 210 qui agissent sur les moyens à vannes 222.1, 222.2.

En effet, les moyens à vannes 222.1, 222.2, i.e. des vannes de contrôle ou analogue, contrôlent le passage du flux de NO/N₂ dans les deux tronçons 201, 202 pour opérer une alimentation alternative de la partie aval du circuit de gaz 200 se trouvant notamment en aval du site de raccordement 203, c'est-à-dire que seul l'un ou l'autre des tronçons 201, 202 peut alimenter ladite partie aval du circuit de gaz 200 mais jamais les deux de manière concomitante, c'est-à-dire en même temps.

Autrement dit, quand le premier moyen à vanne 222.1 est commandé par les moyens de pilotage 210 pour être en position ouverte et ainsi laisser passer du gaz, le second moyen à vanne 222.2 est (commandé) en position fermée et interdit alors tout passage de gaz, et inversement. Toutefois, il est possible que les deux moyens à vannes 222.1, 222.2 soient tous les deux (commandés) en position fermée pour bloquer tout envoi du gaz dans la partie aval du circuit 200, comme expliqué ci-après.

Par ailleurs, on voit que le premier tronçon de circuit 201 et le second tronçon de circuit 202 comprennent chacun une ligne d'échappement à l'atmosphère 204.1, 204.2, à savoir une première 204.1 et une seconde 204.2 ligne d'échappement à l'atmosphère, typiquement des conduits ou analogues. Les lignes d'échappement à l'atmosphère 204.1, 204.2 sont reliées fluidiquement à l'atmosphère via un ou des orifices d'échappement 205, à savoir ici un orifice d'échappement unique 205.

Chaque ligne d'échappement à l'atmosphère 204.1, 204.2 comprend une valve d'échappement 206 pilotée par les moyens de pilotage 210 de manière à contrôler leur ouverture ou leur fermeture, donc autoriser/permettre ou interdire/stopper tout échappement de gaz provenant de l'un ou l'autre des tronçons 201, 202 vers l'atmosphère ambiante. Ces lignes d'échappement à l'atmosphère 204.1, 204.2 sont notamment utilisées lors des phases de purge des tronçons 201, 202 et des tuyaux flexibles qui y sont raccordés, c'est-à-dire des première et seconde lignes d'amenée de gaz 12.1, 12.2, comme expliqué ci-après.

Comme déjà dit, un premier moyen de mesure de pression 251, tel un premier capteur de pression, est agencé sur le premier tronçon de circuit 201, afin de mesurer la pression du gaz, i.e. NO/N₂, au sein du premier tronçon de circuit 201 et, de manière analogue, un second moyen de mesure de pression 252, tel un second capteur de pression, est agencé sur le second tronçon de circuit 202, afin de mesurer la pression du gaz, i.e. NO/N₂, au sein du second tronçon de circuit 202.

Le premier et second moyens de mesure de pression 251, 252, i.e. leurs prises de pression, sont agencés entre les entrées de NO 201.1, 202.1 et les deux moyens à vannes 222.1, 222.2 agencés sur les tronçons de circuit 201, 202, comme visible en Fig. 2, de préférence à proximité des entrées de NO 201.1, 202.1. Ces moyens de mesure de pression 251, 252 fournissent les mesures de pression opérées (i.e. signal ou valeur) aux moyens de pilotage 210, qui les traitent comme détaillé ci-après.

De manière classique, les moyens de pilotage 210 sont reliés électriquement, via des liaisons électriques ou analogues, tels des câbles électriques, aux moyens de mesure de pression 251, 252, aux valves d'échappement 206 et aux moyens à vanne 222.1, 222.2, afin d'assurer les transferts de données, typiquement les mesures, et/ou les pilotages.

L'appareil de délivrance de NO 1 de l'installation 100 comprend par ailleurs une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier. Ceci permet d'alimenter le circuit de gaz interne 200 en oxygène quand cela est nécessaire.

Le ventilateur médical 50, c'est-à-dire un appareil d'assistance respiratoire, fournit un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂).

Le ventilateur médical 50 et l'appareil de fourniture de NO 1 de l'installation 100 sont en communication fluidique avec un circuit respiratoire 20, aussi appelé circuit patient, en particulier avec une ligne d'alimentation en gaz ou branche inspiratoire 21 du circuit respiratoire 20, qui sert à acheminer le flux gazeux vers l'interface respiratoire 40 fournissant le flux gazeux thérapeutique au patient, c'est-à-dire un mélange gazeux final contenant la posologie en NO souhaitée.

Plus précisément, le mélange gazeux final à administrer au patient est formé par mélange du flux à base d'oxygène (e.g. air ou mélange O₂/N₂) provenant du ventilateur médical 50 et du flux contenant le NO, i.e. le mélange gazeux NO/N₂, délivré par l'appareil de délivrance de NO 1.

Pour ce faire, l'appareil de délivrance de NO 1 fournit ou injecte le mélange NO/N₂ dans le circuit respiratoire 20, typiquement dans la branche inspiratoire 21, via un conduit ou une ligne d'injection 23, reliant fluidiquement le circuit de gaz interne de l'appareil de fourniture de NO 1 à un dispositif d'injection 24 agencé sur la ligne d'alimentation en gaz 21.

Le dispositif d'injection 24 est configuré pour opérer un mélange du gaz contenant du NO provenant de l'appareil de délivrance de NO 1 avec le flux de gaz respiratoire contenant de l'O₂ provenant du ventilateur 50 et acheminé par la branche inspiratoire 21 du circuit respiratoire 20, et obtenir un mélange gazeux combiné contenant du NO et de l'oxygène, c'est-à-dire le mélange gazeux final administré au patient.

Plus précisément, le dispositif d'injection 24 comprend une première entrée de gaz alimentée en flux de gaz respiratoire contenant de l'O₂ provenant du ventilateur médical 50, une seconde entrée de gaz alimentée en gaz contenant du NO, i.e. provenant de l'appareil de délivrance de NO 1, et une sortie de gaz fournissant le mélange gazeux combiné contenant du NO et de l'oxygène, obtenu par mélange, au sein du dispositif d'injection 24, du gaz contenant du NO avec le flux de gaz respiratoire contenant de l'O₂.

Autrement dit, le flux de NO/N₂ amené par la ligne d'injection 23 se mélange alors (grâce au dispositif d'injection 24) au flux de gaz à base d'oxygène (> 20% d'O₂), e.g. de l'air ou un mélange oxygène/azote, délivré par le ventilateur médical 50 et véhiculé par la branche inspiratoire 21 du circuit patient 20 de sorte d'obtenir un mélange final, i.e. un mélange combiné, à administrer au patient contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux final NO/N₂/O₂.

La branche inspiratoire 21 du circuit 20 comprend en outre un humidificateur de gaz 30 agencé en aval du dispositif d'injection 24. Il permet d'humidifier le flux de gaz final, e.g. le mélange gazeux combiné NO/N₂/O₂, avant qu'il ne soit administré par inhalation au patient à traiter, au moyen d'une interface respiratoire 40, telle une sonde d'intubation trachéale, un masque respiratoire ou analogue.

Une ligne de récupération des gaz expirés par le patient forme une branche expiratoire 22 du circuit patient 20. Elle est reliée fluidiquement à la branche inspiratoire 21 via une pièce de raccordement 25, telle une pièce en Y.

La branche inspiratoire 21 est, à son extrémité amont, raccordée fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂ fourni par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient. La branche expiratoire 22 peut comprendre un ou plusieurs composants optionnels, par exemple un dispositif d'élimination du CO₂ 35, un filtre ou autre.

Par ailleurs, un capteur de débit 25, par exemple du type à fil chaud, à différentiel de pression ou massique, est agencé sur le circuit respiratoire 20, en particulier sur la branche inspiratoire 21, entre le ventilateur 50 et le dispositif d'injection 24. Le capteur de débit 25 est relié à un port de connexion au capteur 27, de l'appareil de délivrance de NO 1, via une (des) ligne de mesure de débit 26 venant se raccorder audit port de connexion au capteur 27. Il sert à mesurer le débit de gaz délivré par le ventilateur 50, tel de l'air ou N₂/O₂, circulant dans la branche inspiratoire 21, en amont du dispositif d'injection.

Ces mesures de débit opérées par le capteur de débit 25 permettent de contrôler ou réguler plus efficacement la délivrance du flux de NO (i.e. N₂/O₂) par l'appareil de délivrance de NO 1, en particulier le débit de NO, puisque les mesures de débit opérées par le capteur de débit 25 sont retournées, via la ligne de mesure de débit 26 (i.e. câbles électriques ou analogues) et le port de connexion au capteur 27, aux moyens de pilotage 210 à (micro)processeur de l'appareil de délivrance de NO 1, typiquement un (micro)contrôleur, qui traitent ces mesures de débit comme expliqué ci-après et illustré en Fig. 2. Le port de connexion au capteur 27 est relié électriquement aux moyens de pilotage 210 via une ou des liaisons électriques, par exemple des câbles électriques ou analogues.

L'appareil de fourniture de NO 1 de l'installation 100 comprend une carcasse rigide 1.1, par exemple en polymère, comprenant le circuit de gaz interne 200 sur Fig. 2, typiquement des lignes, passages ou conduits de gaz ou analogue, servant à acheminer le flux de gaz à base de NO, i.e. le mélange NO/N₂, provenant des bouteilles de mélange de NO/N₂ 12. Le circuit de gaz interne 200 relie fluidiquement les entrées de gaz 201.1, 202.1 de l'appareil de fourniture de NO 1 à la ligne d'injection 23 de manière à convoyer le flux de gaz à base de NO entre eux.

Dans le mode de réalisation schématisé en Fig. 2, une portion du circuit de gaz interne 200 comprend deux tronçons additionnels de gaz agencés en parallèle, à savoir un tronçon principal 200.1 et un tronçon secondaire 200.2, dit tronçon de secours. Le tronçon principal 200.1 et le tronçon secondaire 200.2 se raccordent fluidiquement l'un à l'autre et au reste du circuit de gaz 200 en des sites de raccordement amont 260 et aval 261 situés, respectivement, en amont et en aval de moyens de contrôle de débit principaux et secondaires 220, 221.

Dans ce cas, en mode de fonctionnement normal, le flux de NO/N₂ transite par le tronçon principal 200.1, alors qu'en cas de dysfonctionnement, par exemple si les moyens de contrôle de débit principaux 220, tel un contrôleur de débit massique ou MFC, sont rendus non-opérationnels ou dysfonctionnels, le flux de NO/N₂ peut transiter par le tronçon de secours 200.2.

Bien entendu, selon un autre mode de réalisation (non montré), le circuit de gaz interne 200 pourrait être configuré différemment, par exemple comprendre une ligne de gaz unique en lieu et place des deux tronçons 200.1, 200.2, laquelle serait utilisée en mode de fonctionnement normal et en mode secours. Toutefois, dans ce mode de réalisation, un dysfonctionnement des moyens de contrôle de débit principaux 220 ne pourrait pas être pris en compte et l'appareil 1 deviendrait alors non-fonctionnel.

D'une façon générale, les moyens de contrôle de débit principaux et secondaires 220, 221, tels des moyens à vanne principaux et secondaires 2200, 2210, schématisés en Fig. 2, i.e. un (ou des) dispositif à vanne(s), par exemple une (ou des) électrovanne proportionnelle pilotée par les moyens de pilotage 210, sont agencés sur le circuit de gaz interne 200, notamment sur les tronçons principal 200.1 et secondaire 200.2, et servent à contrôler ou ajuster le flux gazeux qui y circule en direction de la ligne d'injection 23, c'est-à-dire vers le dispositif d'injection 24 et ce, que ce soit en mode de fonctionnement normal ou en mode de secours.

De préférence, le tronçon principal 200.1 comprend une électrovanne proportionnelle 220 et un capteur de débit additionnel 230, typiquement un contrôleur de débit massique ou MFC, alors que le tronçon secondaire 200.2 comprend une (ou des) électrovanne de type tout ou rien (TOR) 221, de préférence pilotée en mode pulsée. Préférentiellement, les moyens de contrôle de débit principaux et secondaires 220, 221 de l'appareil de fourniture de NO 1 sont commandés, i.e. contrôlés, par les moyens de pilotage 210, c'est-à-dire un (ou des) dispositif de pilotage ou (micro)contrôleur, agencés dans le boitier 1.1 de l'appareil de fourniture de NO 1.

D'une façon générale, les moyens de pilotage 210 de l'appareil 1, tel un contrôleur, comprennent une (des) carte électronique comprenant un (ou plusieurs) microprocesseur(s) 211 mettant en œuvre un ou des algorithmes.

Les moyens de pilotage 210 permettent notamment d'ajuster ou contrôler le débit de gaz à base de NO en pilotant tout ou partie des moyens à vanne 2200, 2210, typiquement d'ouvrir ou fermer une ou des (électro)vannes, pour obtenir un débit de gaz à base de NO, typiquement autoriser ou stopper le débit de gaz.

Bien entendu, les moyens de pilotage 210 permettent en outre d'opérer des calculs et/ou de contrôler ou commander tous les éléments électromécaniques de l'appareil 1, tels que les électrovannes, les affichages ...

En particulier, en fonctionnement, les moyens de pilotage 210 peuvent déterminer le débit de NO à fournir pour obtenir la teneur en NO souhaitée dans le mélange combiné, c'est-à-dire la posologie en NO désirée, à partir notamment de la consigne de teneur en NO réglée et/ou fixée par l'utilisateur, de la composition du mélange gazeux NO/N₂, en particulier de la teneur en NO dans ce mélange gazeux NO/N₂, et d'une (ou des) mesure de débit opérée(s) par le capteur de débit 25 agencé sur la branche inspiratoire 21 et relié par une ligne de mesure de débit 26 au dispositif de fourniture de NO 1, en particulier aux moyens de pilotage 210, via le port de connexion au capteur 27.

Le circuit de gaz interne 200 de l'appareil de fourniture de NO 1 peut aussi comprendre d'autres éléments ou composants, en particulier un (ou des) capteur de pression, un (ou des) capteur de débit additionnel ou débitmètre et/ou des dispositifs à orifice calibré 240 ou autres. Ces autres éléments peuvent être agencés en amont et/ou en aval des moyens de contrôle de débit 220, 221, i.e. des moyens à vanne, par exemple on peut utiliser un capteur de débit additionnel pour déterminer le débit de gaz à base de NO circulant dans tout ou partie du circuit de gaz interne 200, notamment pour s'assurer qu'il est conforme au débit souhaité.

En Fig. 2, on voit que le tronçon principal 200.1 comprend un capteur de débit additionnel 230 agencé en amont des moyens de contrôle de débit 220, tels des moyens à vanne 2200, par exemple une (des) électrovanne, préférentiellement une électrovanne proportionnelle, contrôlant le passage de gaz dans le tronçon principal 200.1. Cet ensemble forme un contrôleur de débit massique (MFC).

Par ailleurs, le tronçon secondaire 200.2 comprend un dispositif à orifice calibré 240 agencé en aval de moyens de contrôle de débit secondaire 221, tels des moyens à vanne secondaire 2210, préférentiellement une (des) électrovanne, contrôlant le débit de passage de gaz dans le tronçon secondaire 200.2.

Avantageusement, l'électrovanne des moyens de contrôle de débit secondaire 221 est du type tout ou rien (TOR), c'est-à-dire pouvant adopter 2 positions « stables », à savoir une position ouverte laissant passer le flux gazeux et une position fermée empêchant toute circulation de flux gazeux.

Par ailleurs, le débitmètre ou capteur de débit additionnel 230 du MFC peut être du type à différentiel de pression, massique ou autre, et coopère avec les moyens de pilotage 210 pour leur fournir des mesures de débit du flux de NO/N₂.

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

L'alimentation électrique de l'appareil de fourniture de NO 1, en particulier des composants nécessitant du courant électrique pour fonctionner, tels les moyens de pilotage 210, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 à l'appareil de fourniture de NO 1. Elle vient se raccorder fluidiquement (en 61) à la ligne d'alimentation en gaz 21, entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue. La ligne de prélèvement de gaz 60 permet de prélever des échantillons de gaz et de les convoyer jusqu'au dispositif de fourniture de NO 1 où ils sont analysés dans un analyseur de gaz interne (non montré), c'est-à-dire au sein d'une ligne de calibration comprenant au moins un capteur, notamment une ou des cellules électrochimiques, relié électriquement aux moyens de pilotage, afin de vérifier leur conformité. En particulier, il convient de vérifier que la composition du gaz final est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, qu'il ne contient pas une teneur en NO₂ trop élevée et que sa teneur en NO correspond à la posologie souhaitée, i.e. dose de NO à administrer par inhalation qui est habituellement choisie par le personnel soignant, i.e. médecin ou analogue. Cette vérification de conformité se fait classiquement au moyen de moyens de mesure dédiés, typiquement des capteurs de NO₂, de NO et d'O₂, par exemple des cellules électrochimiques ou analogues, qui doivent eux-mêmes être calibrés périodiquement, par exemple toutes les semaines. Les moyens de pilotage 210 de l'appareil 1 sont en outre configurés pour récupérer et traiter, i.e. analyser, les signaux provenant des différents capteurs de l'analyseur de gaz, lequel est agencé dans l'appareil 1, et d'agir en réponse à ces signaux, notamment pour opérer une calibration des capteurs.

La valeur de consigne de NO et/ou la teneur en NO dans le mélange gazeux NO/N₂ alimentant l'appareil 1 peuvent être entrées et/ou ajustées et/ou modifiées par l'utilisateur, par exemple via l'IHM, grâce à des moyens de réglage de dose ou analogue, telles des touches, des curseurs ou autres. Préférentiellement, la valeur de consigne de NO et/ou la teneur en NO dans le mélange gazeux NO/N₂ alimentant l'appareil 1 peuvent être mémorisées par les moyens de mémorisation 212 de l'appareil 1.

Selon l'invention, afin d'éviter une interruption de traitement des patients lorsqu'une des bouteilles de NO 10.1, 10.2 est vide, du fait d'un oubli ou d'une non-disponibilité du personnel soignant pour opérer un basculement de l'alimentation de l'appareil de fourniture de NO 1 entre la bouteille vide vers la bouteille pleine, et ce, dans le but d'améliorer la sécurité de traitement des patients traités par administration de NO gazeux, les moyens de pilotage 210 sont configurés pour piloter automatiquement le premier moyen à vanne 222.1 et/ou le second moyen à vanne 222.2 pour contrôler la circulation du gaz dans le premier tronçon de circuit 201 et le second tronçon de circuit 202 en fonction d'une (ou des) valeur de pression-seuil (PS) donnée, comme déjà expliqué ci-avant.

Plus précisément, les moyens de pilotage 210 sont programmés pour autoriser une circulation de gaz, i.e. NO/N₂, au sein du premier tronçon de circuit 201 et simultanément interdire toute circulation de gaz au sein du second tronçon de circuit 202 (et inversement/réciproquement) tant que la pression gazeuse P mesurée par le premier moyen de mesure de pression 251, i.e. une pression instantanée, est supérieure ou égale à une pression-seuil donnée PS, i.e. P ≥ PS, par exemple une pression-seuil PS inférieure ou égale à 4 bar, avantageusement entre 3 et 4 bar. Bien entendu, un autre seuil de pression pourrait être choisi.

La valeur de pression-seuil PS peut être mémorisée par les moyens de mémorisation 212 de l'appareil 1, telle une mémoire informatique, comme une mémoire flash, RAM ou analogue.

En général, lorsqu'une bouteille de gaz 10.1, 10.2 est pleine (avant tout soutirage), la pression de gaz qui y règne est généralement de plus de 150 bar, en général d'au moins 180 bar, et peut aller jusqu'à 300 bar, ou plus.

Lorsque le gaz est soutiré de l'une ou l'autre des bouteilles 10, par exemple de la bouteille 10.1, il subit une réduction de sa pression au sein de moyens de réduction de pression, tels des détendeurs de gaz, agencés en aval du robinet équipant chaque bouteille ou, selon un autre mode de réalisation, intégrés dans ledit robinet (i.e. un RDI), jusqu'à une pression de détente (PD) désirée, typiquement inférieure à 10 bar, par exemple entre 4 et 7 bar, de préférence entre 5 et 6 bars.

La réduction de pression du gaz contenant du NO provenant du premier et/ou du second récipient de NO 10.1, 10.2 jusqu'à la pression de détente (PD) souhaitée, qui est inférieure à 10 bar, est opérée des premier et/ou second moyens de détente 102 ; 102.1, 102.2, tels des détendeurs de gaz, agencés en aval des robinets 101 équipant les récipients de NO 10.1, 10.2.

Le gaz à la pression de détente (PD) ayant traversé les moyens de détente 102 ; 102.1, 102.2, est ensuite acheminé jusqu'à l'appareil 1 par la première ligne d'amenée de gaz 12.1 ou, selon le cas, par la seconde ligne d'amenée de gaz 12.2, en particulier jusqu'à la première entrée de gaz 201.1 du premier tronçon de circuit 201 ou, selon le cas, à la seconde entrée de gaz 202.1 du second tronçon de circuit 202.

Au fur et à mesure de la consommation ou utilisation du gaz, i.e. NO/N₂, la première bouteille 10.1 se vide et la pression du gaz qu'elle fournit tend à diminuer progressivement jusqu'à passer en dessous de la valeur de pression de détente. Le gaz à pression inférieure à la pression de détente (e.g. PD = 10 bar) continue à être acheminé, comme précédemment, jusqu'à l'appareil 1, c'est-à-dire aux tronçons de circuit 201, 202.

Selon l'invention, comme déjà dit, cette décroissance progressive de pression peut être suivie par les moyens de mesure de pression 251, 252 qui transmettent leurs mesures aux moyens de pilotage 210.

Dès lors, dès que la pression gazeuse mesurée P par exemple par le premier moyen de mesure de pression 251 au sein du premier tronçon 201 devient inférieure à la pression-seuil fixée PS, i.e. P < PS, par exemple inférieure à 4 bar, les moyens de pilotage 210 sont configurés pour interrompre toute circulation de gaz au sein du premier tronçon de circuit 201 et par ailleurs autoriser une circulation de gaz au sein du second tronçon de circuit 202, ce qui permet de garantir une continuité de fourniture de NO au circuit interne 200 de l'appareil de délivrance de NO 1.

Le gaz, i.e. NO/N₂, est alors fourni, de la même façon, par la deuxième bouteille de gaz 10.2, en étant détendu en sortie de la deuxième bouteille 10.2 jusqu'à la pression de détente (PD), typiquement inférieure à 10 bar, ce qui engendre à nouveau une vidange progressive de cette deuxième bouteille 10.2 avec, là encore, diminution progressive de la pression gazeuse, notamment dans le deuxième tronçon 202, qui va passer sous la pression de détente (PD) lorsque la bouteille 10.2 sera proche d'être vide.

Ceci est surveillé, comme expliqué ci-avant, par les moyens de pilotage 210 qui reçoivent les mesures de pression P, i.e. pression instantanée, provenant du second moyen de mesure de pression 252 agencé dans le second tronçon 202.

Là encore, lorsque la pression gazeuse mesurée P par le second moyen de mesure de pression 252 au sein du premier tronçon 201 devient inférieure à la pression-seuil donnée PS, i.e. P < PS, par exemple inférieure à une pression-seuil donnée PS égale à 4 bar, les moyens de pilotage 210 sont configurés pour interrompre toute circulation de gaz au sein du second tronçon de circuit 202 et par ailleurs autoriser une circulation de gaz au sein du premier tronçon de circuit 201.

Autrement dit, grâce à la présente invention, il se produit un basculement automatique d'un tronçon à l'autre 201, 202, et ce de manière alternative, en fonction de la pression de gaz qui y règne et de la pression-seuil donnée PS.

La valeur de pression-seuil donnée PS, par exemple 4 bar, peut être fixée une fois pour toute et mémorisée ou, selon le cas, être modifiable, notamment via l'IHM de l'appareil de NO 1.

Avantageusement, la valeur de pression-seuil donnée PS est comprise entre 3 et 6 bar, de préférence entre 3.5 et 5 bar, de préférence encore entre 3 et 4 bar. Une telle valeur de pression correspond à une bouteille qui n'est pas encore totalement vide mais qui le sera prochainement. Cette pression résiduelle permet d'assurer un basculement d'approvisionnement en gaz de la bouteille (quasi-)vide vers une bouteille à pression supérieure, typiquement une bouteille (quasi)pleine en toute sécurité pour le patient, sans interruption de la fourniture de NO au patient pendant la durée nécessaire à ce basculement, y compris pendant le temps de purge.

Préférentiellement, après interruption de la circulation du gaz par exemple dans le premier tronçon 201 (ou alternativement le second tronçon 202) alimenté par la bouteille de gaz 10.1 (quasi)vide mais avant autorisation de la circulation du gaz dans l'autre tronçon, i.e. le second tronçon 202, (ou alternativement le premier tronçon 201) qui est alors alimenté par l'autre bouteille de gaz 10.2, i.e. la bouteille pleine, on opère une purge du second tronçon 202 et préférentiellement d'au moins une partie de la ligne d'alimentation 12.2, tel un conduit flexible, reliant la bouteille de gaz 10.2, pleine au second tronçon 202 afin de les débarrasser d'impuretés gazeuses pouvant s'y trouver, en particulier tout composé toxique de type NO₂ susceptible de s'y trouver résultant d'une oxydation de molécules de NO par des molécules d'oxygène.

Bien entendu, on procède de même pour purger le premier tronçon 201 avant d'y envoyer du gaz, lorsque la seconde bouteille de gaz 10.2 est quasi-vide, c'est-à-dire lorsque la pression mesurée dans le second tronçon 202 devient inférieure à la pression-seuil PS, e.g. de 3 à 4 bar.

Dans tous les cas, la purge s'opère avec une partie du mélange NO/N₂ provenant des récipients de gaz 10.1, 10.2. Pour opérer la purge de l'un ou l'autre des tronçons 201, 202, et du conduit flexible qui lui est associé, i.e. auquel il est raccordé fluidiquement, on utilise la première 204.1 ou la seconde 204.2 ligne d'échappement à l'atmosphère en fonction du tronçon à purger, à savoir le premier tronçon de circuit 201 ou le second tronçon de circuit 202, respectivement, pour évacuer vers l'atmosphère, via l'orifice d'échappement 205, le gaz qui s'y trouve et d'au moins une partie des impuretés gazeuses pouvant s'y trouver, telles les espèces NO₂ toxiques.

Cette purge se fait par balayage gazeux avec le mélange NO/N₂ provenant des récipients de gaz 10.1, 10.2, lequel balayage gazeux entraine les impuretés gazeuses, telles les espèces NO₂ toxiques, présentes dans le tronçon 201, 202 à purger et dans le conduit flexible qui lui associé, et le flux de gaz de purge ainsi créé est évacué vers l'atmosphère extérieure, via l'orifice d'échappement 205. Ce flux de purge contient donc essentiellement de l'azote, du NO et des impuretés éventuelles comme le NO₂.

Les moyens de pilotage 210 commandent la (ou les) valve d'échappement 206 agencée(s) sur la première 204.1 et/ou la seconde 204.2 ligne d'échappement reliée au tronçon 201, 202 à purger de manière à contrôler son ouverture (ou sa fermeture), pour autoriser (ou interdire) l'échappement du gaz provenant du tronçon 201, 202 à purger, et généralement de la ligne d'alimentation en gaz 12.1, 12.2 qui y est reliée.

Ceci permet d'accroître la sécurité pour le patient en éliminant des espèces toxiques éventuellement présentes dans le tronçon non utilisé et son conduit d'alimentation.

De préférence, la purge pendant une durée de purge donnée, typiquement comprise entre 10 et 120 secondes, typiquement d'au moins 30 secondes. La durée de purge est mémorisée, par exemple par les moyens de mémorisation de l'appareil 1.

Une installation d'administration de gaz 100 peut être utilisée pour administrer par inhalation du monoxyde d'azote (NO), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

1. Installation (100) de fourniture d'un mélange gazeux contenant du NO à un patient comprenant :
A) un premier et un second récipient de NO (10.1, 10.1) contenant un gaz contenant du NO à une pression donnée,
B) un appareil de délivrance de NO (1) pour fournir le gaz contenant du NO comprenant :
- des moyens de pilotage (210),
- un circuit de gaz (200) comprenant :
▪ un premier tronçon de circuit (201) comprenant une première entrée de gaz (201.1) configurée pour y raccorder fluidiquement le premier récipient de NO (10.1) contenant le gaz contenant du NO, et
▪ un second tronçon de circuit (202) comprenant une seconde entrée de gaz (202.1) configurée pour y raccorder fluidiquement le second récipient de NO (10.2) contenant le gaz contenant du NO,
- un premier moyen de mesure de pression (251), agencé sur le premier tronçon de circuit (201), configuré pour mesurer la pression au sein du premier tronçon de circuit (201),
- un second moyen de mesure de pression (252), agencé sur le second tronçon de circuit (202), configuré pour mesurer la pression au sein du second tronçon de circuit (202),
- un premier moyen à vanne (222.1), agencé sur le premier tronçon de circuit (201), piloté par les moyens de pilotage (210) pour contrôler le flux de gaz au sein du premier tronçon de circuit (201), et
- un second moyen à vanne (222.2), agencé sur le second tronçon de circuit (202), piloté par les moyens de pilotage (210) pour contrôler le flux de gaz au sein du second tronçon de circuit (202),
C) des premier et second moyens de détente (102 ; 102.1, 102.2) agencés en amont de l'appareil de délivrance de NO (1) et configurés pour opérer une réduction de pression du gaz contenant du NO provenant des premier et second récipient de NO (10.1, 10.2) jusqu'à une pression de détente (Pd) donnée inférieure à 10 bar, et
D) une première ligne d'amenée de gaz (12.1) et une seconde ligne d'amenée de gaz (12.2), respectivement, pour acheminer le gaz contenant du NO ayant traversé les moyens de détente (102 ; 102.1, 102.2) jusqu'à la première entrée de gaz (201.1) du premier tronçon de circuit (201) et à la second entrée de gaz (202.1) du second tronçon de circuit (202), respectivement,
**caractérisé en ce que** les moyens de pilotage (210) de l'appareil de délivrance de NO (1) sont configurés pour piloter le premier moyen à vanne (222.1) et/ou le second moyen à vanne (222.2) pour :
iii) autoriser une circulation de gaz au sein de l'un des premier et second tronçons de circuit (201, 202) et simultanément interdire toute circulation de gaz au sein de l'autre desdits premier et second tronçons de circuit (201, 202) tant que la pression (P) mesurée par le premier ou le second moyen de mesure de pression (251, 252) est supérieure ou égale à une pression-seuil donnée (PS), i.e. P ≥ PS, et
iv) lorsque la pression mesurée (P) par le premier ou le second moyen de mesure de pression (251, 252) devient inférieure à la pression-seuil donnée (PS), i.e. P < PS :
a) interrompre toute circulation de gaz au sein dudit premier ou second tronçon de circuit (201, 202) au sein duquel du gaz circule et
b) autoriser une circulation de gaz au sein de l'autre desdits premier ou second tronçon de circuit (202) au sein duquel la circulation de gaz était interdite,
où ladite pression-seuil donnée (PS) est inférieure à la pression de détente (PD).

2. Installation selon la revendication 1, **caractérisée en ce que** le premier tronçon de circuit (201) et le second tronçon de circuit (202) de l'appareil (1) viennent se raccorder l'un à l'autre en un site de raccordement (203) du circuit de gaz (200) situé en aval des premier et second moyens à vanne (222.1, 222.2).

3. Installation selon la revendication 1, **caractérisée en ce que** la première entrée de gaz (201.1) de l'appareil (1) est configurée pour être raccordée fluidiquement au premier récipient de NO (10.1) par l'intermédiaire de la première ligne d'amenée de gaz (12.1) et la seconde entrée de gaz (202.1) de l'appareil (1) est configurée pour être raccordée fluidiquement au second récipient de NO (10.2) par l'intermédiaire de la seconde ligne d'amenée de gaz (12.2).

4. Installation selon la revendication 3, **caractérisée en ce que** la première ligne d'amenée de gaz (12.1) et la seconde ligne d'amenée de gaz (12.2) de l'appareil (1) comprennent des tuyaux flexibles.

5. Installation selon la revendication 1, **caractérisée en ce que** :
- une première ligne d'échappement à l'atmosphère (204.1) comprenant une première valve d'échappement (206.1), est raccordée fluidiquement au premier tronçon de circuit (201), et
- une seconde ligne d'échappement à l'atmosphère (204.2) comprenant une seconde valve d'échappement (206.2), est raccordée fluidiquement au second tronçon de circuit (202),
et dans lequel lesdites première et seconde valves d'échappement (206.1) sont pilotées par les moyens de pilotage (210).

6. Installation selon la revendication 5, **caractérisée en ce que** la première ligne d'échappement à l'atmosphère (204.1) et la seconde ligne d'échappement à l'atmosphère (204.2) communiquent avec l'atmosphère via un orifice d'échappement unique (205).

7. Installation selon les revendications 1 et 5, **caractérisée en ce que** les moyens de pilotage (210) sont en outre configurés pour :
- piloter la seconde valve d'échappement (206.2) pour opérer une purge du second tronçon de circuit (202), avant d'autoriser une circulation de gaz provenant du second récipient de NO (10.2) au sein dudit second tronçon de circuit (202) ou,
- alternativement, piloter la première valve d'échappement (206.1) pour opérer une purge du premier tronçon de circuit (201), avant d'autoriser une circulation de gaz provenant du premier récipient de NO (10.1) au sein dudit premier tronçon de circuit (201).

8. Installation selon les revendications 3 et 6, **caractérisée en ce que** les moyens de pilotage (210) sont en outre configurés pour opérer une purge du premier ou du second tronçon de circuit (201, 202) et simultanément d'au moins une partie de la première ou de la seconde ligne d'amenée de gaz (12.1, 12.2) reliée audit premier ou second tronçon de circuit (201, 202) soumis à purge.

9. Installation selon la revendication 1, **caractérisée en ce que** la pression de détente (PD) est comprise entre 4 et 7 bar, de préférence entre 4 et 6 bar, préférentiellement encore entre 5 et 6 bar.

10. Installation selon la revendication 1, **caractérisée en ce que** la pression-seuil donnée (PS) est comprise entre 2 et 5 bar, de préférence entre 2 et 4 bar préférentiellement encore entre 3 et 4 bar.

11. Installation selon l'une des revendications 1 ou 10, **caractérisée en ce que** la pression-seuil donnée (PS) est mémorisée par des moyens de mémorisation (212) de l'appareil (1).

12. Installation selon la revendication 7, **caractérisé en ce que** :
- les moyens de pilotage (210) sont en outre configurés pour opérer une purge pendant une durée de purge donnée, de préférence une durée de purge comprise entre 10 secondes et 120 secondes, et/ou
- la purge comprend un balayage gazeux avec le mélange gazeux contenant du NO, typiquement le mélange NO/N₂, provenant du premier ou du second récipient de NO (10.1, 10.1).

13. Installation selon la revendication 1, **caractérisée en ce qu'**elle comprend en outre :
- un ventilateur médical (50) configuré pour fournir un flux de gaz respiratoire contenant de l'O₂, et
- un circuit respiratoire (20 ; 21) comprenant un dispositif d'injection (24) configuré pour opérer un mélange du gaz contenant du NO provenant de l'appareil de délivrance de NO (1) avec le flux de gaz respiratoire contenant de l'O₂ fourni par le ventilateur médical (50), et obtenir un mélange gazeux combiné contenant du NO et de l'oxygène.

14. Installation selon la revendication 1, **caractérisée en ce que** le premier et le second récipient de NO (10.1, 10.1) contiennent un mélange gazeux NO/N₂ contenant entre 100 et 2000 ppmv de NO, le reste étant de l'azote (N₂).

15. Installation selon la revendication 1, **caractérisée en ce que** :
- le premier et le second récipient de NO (10.1, 10.1) sont équipés chacun d'un robinet de distribution de gaz (101), et
- les moyens de détente (102 ; 102.1, 102.2) sont intégrés aux robinet de distribution de gaz (101) ou agencés en aval des robinet de distribution de gaz (101) équipant les premier et second récipients de NO (10.1, 10.1).
